# EUROPÄISCHE PATENTSCHRIFT

(11) **EP 0 906 277 B1**
(45) Veröffentlichungstag und Bekanntmachung des Hinweises auf die Patenterteilung: **12.09.2001**
(21) Anmeldenummer: 97929183.8
(22) Anmeldetag: 13.06.1997
(51) Int. Cl.: C07D 201/16, C07D 223/10

(54) **VERFAHREN ZUR REINIGUNG VON EPSILON-CAPROLACTAM**
METHOD OF PURIFYING EPSILON-CAPROLACTAM
PROCEDE DE PURIFICATION DE EPSILON-CAPROLACTAME

(30) Priorität: 13.06.1996 DE 19623662
(43) Veröffentlichungstag der Anmeldung: 07.04.1999
(73) Patentinhaber: BASF AKTIENGESELLSCHAFT, 67056 Ludwigshafen (DE)
(72) Erfinder: FUCHS, Eberhard, D-67227 Frankenthal (DE)
(74) Vertreter: Kinzebach, Werner, Dr.
(86) Internationale Anmeldenummer: EP9703098
(87) Internationale Veröffentlichungsnummer: WO9747596

(56) Entgegenhaltungen:
- EP-A- 0 010 271
- EP-A- 0 138 241
- EP-A- 0 635 487
- EP-A- 0 641 778
- CH-A- 326 165
- FR-A- 1 087 136
- FR-A- 1 334 780
- GB-A- 944 307
- DATABASE WPI Week 7703 Derwent Publications Ltd., London, GB; AN 77-04563y XP002040926 & JP 51 138 690 A (UBE IND. LTD.) , 30.November 1976

## Beschreibung

Die vorliegende Erfindung betrifft ein Verfahren zur Reinigung von ε-Caprolactam.

ε-Caprolactam ist ein wichtiges Ausgangsprodukt für die Herstellung von Polyamiden (Nylon 6). Die industrielle Herstellung von ε-Caprolactam kann auf verschiedenen Wegen erfolgen. In den meisten Fällen erfolgt die Herstellung durch Beckmann-Umlagerung des Cyclohexanonoxims (K. Weissermel, H.J. Arpe, Industrielle Organische Chemie 4. Aufl., S. 272ff.). In einem alternativen Verfahren wird ausgehend von Toluol via Benzoesäure Cyclohexancarbonsäure hergestellt, welche mit Nitrosyl-Schwefelsäure zum ε-Caprolactam umgelagert wird. In weiteren Verfahren werden ω-Aminocapronsäure-Derivate, beispielsweise 6-Aminocapronsäureester (EP-A 376 123) oder 6-Aminocapronitril (EP 659 741), in Gegenwart geeigneter, in der Regel saurer Katalysatoren, zu E-Caprolactam cyclisiert.

Bei allen Verfahren zur Herstellung von ε-Caprolactam fallen Nebenprodukte an, deren Art und Menge vom Verfahrensprinzip, von der Eduktqualität aber auch von den Verfahrensparametern abhängen. Andererseits werden an die Reinheit des ε-Caprolactams, insbesondere bei der Faserherstellung, hohe Ansprüche gestellt. Aus diesem Grunde ist für jedes Herstellungsverfahren ein eigenes optimiertes Reinigungsverfahren notwendig. Die verschiedenen Reinigungsverfahren sind beispielsweise im Process Economics Program Report No. 41 B, Caprolactam and Nylon 6, March 1988, S. 69ff, zitiert.

Diese Reinigungsverfahren bestehen in der Regel aus kombinierten Extraktions-, Destillations- und/oder Kristallisationsverfahren. Stärker verunreinigte Caprolactam-Fraktionen, beispielsweise Rückstände der Caprolactam-Reinigung, werden oftmals einer katalytischen Hydrierung unterworfen. Nach Entfernung des Katalysators erfolgt in der Regel eine destillative Aufarbeitung oder die Rückführung in den Reinigungskreislauf. Im Falle einer katalytischen Suspensionshydrierung des rohen ε-Caprolactams mit Raney-Nickel (EP-A-138 241, JP-A-60-21145) bereitet die Abtrennung des Katalysators Probleme. Bei Hydrierung des rohen ε-Caprolactams an Festbettkatalysatoren (DE-A-1004616, DD-A-75083) ist mit einer im Laufe der Zeit nachlassenden Aktivität oder Vergiftung der Katalysatoren zu rechnen.

Die EP-A-0 635 487 offenbart ein Verfahren zur Reinigung von ε-Caprolactam durch Hydrierung eines Wasser-ε-Caprolactam-Gemisches mit Wasserstoff in Anwesenheit eines heterogenen Katalysators. Dabei bringt man das ε-Caprolactam-Wasser-Gemisch zuerst mit gasförmigem Wasserstoff in Kontakt, wobei sich der Wasserstoff in dem ε-Caprolactam-Wasser-Gemisch löst, und anschließend bringt man das Gemisch mit einem Hydrierungskatalysator in Kontakt.

Die EP-A-0 641 778 offenbart ein Verfahren zur Herstellung eines hochreinen ε-Caprolactams durch Behandlung eines rohen ε-Caprolactams mit Wasserstoff bei 100°C bis 200°C in Anwesenheit eines Hydrierungskatalysators.

Die FR-A-1 334 780 beschreibt ein Verfahren zur Reinigung von Lactamen mit aktiviertem Wasserstoff in Anwesenheit saurer Substanzen. Der aktivierte Wasserstoff wird dabei katalytisch oder in naszierender Form erzeugt.

Aufgabe der vorliegenden Erfindung war die Bereitstellung eines universell einsetzbaren Reinigungsverfahrens für ε-Caprolactam, das mit geringem Aufwand durchgeführt werden kann.

Diese Aufgabe konnte überraschenderweise durch Verwendung eines komplexen Hydrids des Bors gelöst werden.

Gegenstand der vorliegenden Erfindung ist daher ein Verfahren zur Reinigung von ε-Caprolactam, dadurch gekennzeichnet, dass man das zu reinigende ε-Caprolactam mit einem komplexen Borhydrid in Abwesenheit saurer Substanzen behandelt.

Als komplexe Hydride des Bors kommen für das erfindungsgemäße Verfahren insbesondere Natriumborhydrid, Lithiumborhydrid, Kaliumborhydrid, Calciumborhydrid und Natriumcyanoborhydrid in Frage.

Die eingesetzte Menge an Hydrid-Wasserstoff hängt naturgemäß von der Konzentration der Verunreinigungen im ε-Caprolactam ab. Sie wird so gewählt, daß ein Überschuß an Hydrid-Wasserstoff, bezogen auf die zu reduzierenden Verunreinigungen, vorliegt. Ein 1,5 bis fünffacher Überschuß, bezogen auf die stöchiometrisch benötigte Hydridmenge, wird bevorzugt.

Es hat sich gezeigt, daß, um eine ausreichende Reaktionsgeschwindigkeit beim erfindungsgemäßen Reinigungsverfahren von ε-Caprolactam zu erzielen, bei Verwendung der Borhydride der Reaktionsmischung 10 bis 50 Gew.-% Wasser, bezogen auf rohes ε-Caprolactam, zuzusetzen.

In einer bevorzugten Ausführungsform wird die Reaktion in Gegenwart von 0,5 bis 5 Mol-%, insbesondere 1 bis 4 Mol-%, NaBH₄ und 10 bis 50 Gew.-% Wasser, jeweils bezogen auf rohes ε-Caprolactam, durchgeführt. Dabei kann Natriumborhydrid in fester Form sowie als handelsübliche wäßrige Lösung verwendet werden.

Die Reaktion wird vorzugsweise bei Temperaturen im Bereich von 10 bis 150°C, insbesondere 20 bis 100°C, durchgeführt. Die Reaktionsdauer liegt im Bereich von 0,5 h bis 200 h, vorzugsweise im Bereich von 1 h bis 100 h.

Nach der Reaktion werden bei leicht vermindertem Druck Wasser und die reduzierten Verunreinigungen abdestilliert. Auch ist es möglich, bereits während der Reaktion durch Anlegen eines leichten Unterdrucks kontinuierlich Wasser und die Verunreinigungen abzudestillieren. Im Anschluß hieran wird das Reaktionsgemisch einer konventionellen Destillation im Vakuum (0,5-8 mm Hg) unterworfen. Hierbei destilliert reines ε-Caprolactam mit einer UV-Kennzahl < 10 (s.u.) über.

Das erfindungsgemäße Verfahren eignet sich zur Reinigung von ε-Caprolactam, das durch eines der gängigen Herstellungsverfahren hergestellt worden ist. Vorzugsweise eignet sich dieses Verfahren zur Reinigung von ε-Caprolactam, welches durch Cyclisierung von ω-Aminocapronsäurederivaten, beispielsweise ω-Aminocapronsäure, ω-Aminocapronsäureamid, ω-Aminocapronsäureester und ω-Aminocapronsäurenitril, hergestellt wurde.

### Beispiele:

Reinigung eines durch Cyclisierung von ω-Aminocapronsäurenitril hergestellten Caprolactams.

Die Reinheit des E-Caprolactams wird über die UV-Kennzahl ermittelt. Diese ist definiert als Summe der Extinktionen einer 50 Gew.-%igen wäßrigen Caprolactamlösung gemessen zwischen 280 Nanometern und 400 Nanometern im Abstand von 10 Nanometern in einer Küvette mit einer Schichtdicke von d = 5 cm. Die UV-Kennzahl des gereinigten ε-Caprolactams sollte den Wert 10 nicht überschreiten.

### Beispiel 1

### Herstellung des ε-Caprolactams nach DE-A 43 396 48

100 Gewichtsteile ω-Aminocapronsäurenitril wurden in 1000 Gewichtsteilen Ethanol und 30 Gewichtsteilen Wasser gelöst und bei 220°C mit einer Verweilzeit von 12 min. über ein Festbett aus Titanoxid (Anatas) geführt. Anschließend wurde das Lösungsmittel abdestilliert. Das so erhaltene rohe Caprolactam wurde in der folgenden Reinigungsstufe eingesetzt.

### Reinigung des rohen ε-Caprolactams

70 Gewichtsteile Rohcaprolactam und 30 Gewichtsteile Wasser wurden in einem Rührkessel, ausgerüstet mit Destillationsbrücke, mit 1 Gew.-% Natriumborhydrid (bezogen auf rohes ε-Caprolactam) versetzt. Nach 100 h wurde das Reaktionsgemisch fraktioniert destilliert. Hierbei ging ε-Caprolactam bei 119°C (2 mbar) mit einer UV-Kennzahl von 3,1 über.

### Beispiel 2

Rohcaprolactam, hergestellt durch Beckmannumlagerung (siehe Progress Economics Program Report (SRI-Report) N° 41 B, Caprolactam and Nylon 6, March 1988), wurde unter Zusatz von 0,4 Gew.-% NaBH₄ analog Beispiel 1 umgesetzt. Nach 24 h wurde das Reaktionsgemisch fraktioniert destilliert. Bei 125°C (3 mbar) ging ε-Caprolactam mit einer UV-Kennzahl von 3,5 über.

## Patentansprüche

1. Verfahren zur Reinigung von ε-Caprolactam, **dadurch gekennzeichnet, daß** man das zu reinigende ε-Caprolactam mit einem komplexen Borhydrid in Abwesenheit saurer Substanzen behandelt.

2. Verfahren nach Anspruch 1, **dadurch gekennzeichnet, daß** das komplexe Hydrid ausgewählt ist unter NaBH₄, LiBH₄, KBH₄, Ca(BH₄)₂ und NaBH₃CN.

3. Verfahren nach einem der vorhergehenden Ansprüche, **dadurch gekennzeichnet, daß** man 2 bis 20 Mol-% hydridischen Wasserstoff, bezogen auf das zu reinigende ε-Caprolactam, einsetzt.

4. Verfahren nach einem der Ansprüche 1 bis 3, **dadurch gekennzeichnet, daß** man das ε-Caprolactam mit 0,5 bis 5 Mol-% NaBH₄ und 10 bis 50 Gew.-% Wasser, bezogen auf das zu reinigende ε-Caprolactam, behandelt.

## Claims

1. A process for purifying ε-caprolactam, which comprises treating the ε-caprolactam to be purified with a complex borohydride in the absence of acidic substances.

2. A process as claimed in claim 1, wherein the complex hydride is selected from the group consisting of NaBH₄, LiBH₄, KBH₄, Ca(BH₄)₂ and NaBH₃CN.

3. A process as claimed in either of the preceding claims, wherein from 2 to 20 mol% of hydridic hydrogen is used, based on the ε-caprolactam to be purified.

4. A process as claimed in any of claims 1 to 3, wherein the ε-caprolactam is treated with from 0.5 to 5 mol% of NaBH₄ and from 10 to 50 % by weight of water, based on the ε-caprolactam to be purified.

## Revendications

1. Procédé de purification de ε-caprolactame, **caractérisé en ce que** l'on traite le ε-caprolactame à purifier au moyen d'un hydrure de bore complexe, en l'absence de substances acides.

2. Procédé selon la revendication 1, **caractérisé en ce que** l'hydrure complexe est choisi parmi NaBH₄, LiBH₄, KBH₄, Ca(BH₄)₂ et NaBH₃CN.

3. Procédé selon l'une des revendications qui précèdent, **caractérisé en ce que** l'on met en oeuvre de 2 à 20% molaires d'hydrogène hydrique, par rapport au ε-caprolactame à purifier.

4. Procédé selon l'une des revendications 1 à 3, **caractérisé en ce que** l'on traite le ε-caprolactame avec 0,5 à 5% molaires de NaBH₄ et 10 à 50% en poids d'eau, par rapport au ε-caprolactame à purifier.
